# FASCICULE DE BREVET EUROPEEN

(11) **EP 1 427 721 B1**
(45) Date de publication et mention de la délivrance du brevet: **23.01.2008**
(21) Numéro de dépôt: 02774562.9
(22) Date de dépôt: 02.09.2002
(51) Int. Cl.: C07D 401/06, C07D 213/55

(54) **PROCEDE DE SYNTHESE DE 5-(3-PYRIDYLMETHYLENE)-IMIDAZOLIDINE-2,4-DIONE**
VERFAHREN ZUR SYNTHESE VON 5-(3-PYRIDYLMETHYLEN)-IMIDAZOLIDIN-2,4-DIONEN
METHOD FOR SYNTHESIS OF 5-(3-PYRIDYLMETHYLENE)-IMIDAZOLIDINE-2,4-DIONE

(30) Priorité: 05.09.2001 FR 0111637
(43) Date de publication de la demande: 16.06.2004
(73) Titulaire: SOLVAY (Société Anonyme), 1050 Bruxelles (BE)
(72) Inventeur: CALLENS, Roland, B-1850 Grimbergen (BE)
(74) Mandataire: Jacques, Philippe
(86) Numéro de dépôt international: PCT/EP2002/009886
(87) Numéro de publication internationale: WO 2003/022834

(56) Documents cités:
- EP-A- 1 083 170
- DE-A- 4 434 293
- F. ZYMALKOWSKI: "Über die Synthese von Pyridyl-alaninen" ARCHIV DER PHARMAZIE., vol. 291, no. 9, 1958, pages 436-441, XP008002324 VCH VERLAGSGESELLSCHAFT MBH, WEINHEIM., DE ISSN: 0365-6233
- C. DÖBLER ET AL: "Unusual aminoacids. Asymmetric synthesis of 3- and 4-pyridylalanines" TETRAHEDRON: ASYMMETRY, vol. 7, no. 1, 1996, pages 117-125, XP002196132 OXFORD GB
- J.J. BOZELL ET AL: "Transition-metal-assisted asymmetric synthesis of amino acid analogues. A new synthesis of optically pure D- and L- pyridylalanines" JOURNAL OF ORGANIC CHEMISTRY, vol. 56, no. 7, 1991, pages 2584-2587, XP002196133 EASTON US
- MÜLLER; BAYER: 'Methoden der organischen chemie', vol. IV/1C, 1980, GEORG THIEME VERLAG STUTTGART, STUTTGART page 159,188 Band IV/1c pages 159, 188

## Description

La présente invention concerne un procédé pour la synthèse de (3-pyridyl)-alanine. La D-(3-pyridyl)-alanine peut être utilisée comme constituant de peptides, en particulier dans des antagonistes de LHRH.

La demande de brevet EP-1083170-A1 stipule que la D-(3-pyridyl)-alanine peut être obtenue par réaction enzymatique de 5-(3-pyridylméthyl)-imidazolidine-2,4-dione avec des D-hydantoïnases. Toutefois, cette référence ne décrit pas la synthèse de 5-(3-pyridylméthyl)-imidazolidine-2,4-dione. Zymalkowski Arch. Pharm., 291(9), p. 436-441 décrit une formule générale correspondant au 5-(3-pyzidylméthyl)-imidazolidine-2,4-dione comme intermédiaire (non isolé) de la réaction de 5-(3-pyridylméthylène)-imidazolidine-2,4-dione avec du sulfure d'ammonium,

Il était dès lors souhaitable de mettre à disposition un procédé efficace et industrialisable de synthèse de (3-pyridyl)-alanines, en particulier la D-(3-pyridyl)-alanine, comprenant un procédé efficace et industrialisable de synthèse du 5-(3-pyridylméthyl)-imidamlidine-2,4-dione.

L'invention concerne dès lors un procédé de synthèse de (3-pyridyl)-alanine dans lequel
(a) on fabrique du 5-(3-pyridylméthyl)-imidazolidine-2,4-dione par une réaction d'hydrogénation de 5-(3-pyridylméthylène)-imidazolidine-2,4-dione ;
(b) on soumet du 5-(3-pyridylméthyl)-imidazolidine-2,4-dione obtenu à une réaction d'hydrolyse avec une hydantoïnase suivie d'une réaction de décarbamatation.

Il a été trouvé, de manière surprenante, que l'étape (a) du procédé selon l'invention permet de convertir, de façon sélective et avec un rendement élevé le 5-(3-pyridylméthylène)-imidazolidine-2,4-dione en 5-(3 pyridylméthyl)-imidazolidine-2,4-dione. Il est en particulier possible de limiter, voire éviter, la saturation du système aromatique du cycle pyridinique. Le procédé selon l'invention est utilisable pour la fabrication de quantités industrielles de 5-(3-pyridylmëthyl)-imidazolidine-2,4-dione pouvant être utilisés pour la fabrication de (3-pyridyl)-alanines, en particulier de D-(3-pyridyl)-alanine.

Dans le procédé selon l'invention, la réaction d'hydrogénation est de préférence effectuée en phase liquide. Dans ce cas, on dissout avantageusement le 5-(3-pyridylméthylène)-imidazolidine-2,4-dione dans une solution aqueuse d'acide de préférence d'acides minéraux qui peut être mélangé avec un solvant choisi parmi les alcools aliphatiques comprenant de préférence de 1 à 3 atomes de carbone. Un système de solvant particulièrement préféré comprend du méthanol et une solution aqueuse d'acide chlorhydrique.

La réaction d'hydrogénation est effectuée en présence d'un catalyseur d'hydrogénation. Le catalyseur d'hydrogénation est avantageusement choisi parmi les métaux du groupe VIII du Tableau périodique des éléments (IUPAC 1970). On citera en particulier un catalyseur comprenant au moins un métal choisi parmi le palladium, le platine et le rhodium. Un catalyseur comprenant du palladium est préféré. Il a été trouvé qu'un catalyseur d'hydrogénation choisi parmi les métaux du groupe VIII et en particulier un catalyseur comprenant du palladium permet une hydrogénation particulièrement sélective de la double-liaison non-aromatique dans le 5-(3-pyridylméthylène)-imidazolidine-2,4-dione.

Le catalyseur d'hydrogénation est souvent un catalyseur supporté. Des supports utilisables sont choisis par exemple parmi l'alumine, la silice et le charbon actif. Un catalyseur supporté sur charbon actif donne de bons résultats.

Lorsque le catalyseur d'hydrogénation est un catalyseur supporté comprenant un métal du groupe VIII, la teneur en métal est généralement d'au moins 0, 1 % en poids par rapport au poids total du catalyseur. Souvent, la teneur en métal est supérieure ou égale à 1 % en poids. De préférence la teneur en métal est supérieure ou égale à 5% en poids. La teneur en métal est généralement d'au plus 50% en poids par rapport au poids total du catalyseur.

De manière tout particulièrement préférée, le catalyseur est du palladium supporté de préférence sur un support tel que décrit ci-avant et présentant de préférence une teneur en métal telle que décrite ci-avant.

Dans la réaction d'hydrogénation, la température de la réaction est généralement d'au moins -10°C. Souvent, la température de la réaction est d'au moins 0°C. De préférence, cette température est d'au moins 20°C. La température de la réaction est généralement d'au plus 100°C. Souvent, la température de la réaction est d'au plus 60°C. De préférence, cette température est d'au plus 50°C. Une température d'au plus 40°C est tout particulièrement préférée.

Dans la réaction d'hydrogénation, la pression de la réaction est généralement d'au moins 1 bar absolu. De préférence, la pression est d'au moins 1,5 bar. La pression de la réaction d'hydrogénation est généralement d'au plus 10 bar absolu. De préférence, la pression est d'au plus 5 bar. De manière particulièrement préférée, elle est d'au plus 3 bar.

Dans le procédé selon l'invention, on utilise de l'hydrogène à titre de réactif d'hydrogénation. Dans ce cas, les valeurs de pression de la réaction d'hydrogénation mentionnées ci-avant correspondent, en général, à la pression d'hydrogène.

Le rapport molaire entre l'hydrogène et le 5-(3-pyridylméthylène)-imidazolidine-2,4-dione est généralement supérieur ou égal à 1. Ce rapport est généralement d'au plus 100. De préférence ce rapport est d'au plus 10

Dans la réaction d'hydrogénation, la concentration du 5-(3-pyridylméthylène)-imidazolidine-2,4-dione dans le milieu réactionnel est généralement d'au moins 5% en poids par rapport au poids total du milieu réactionnél. Souvent, cette concentration est d'au moins 10% en poids. De préférence, la concentration est d'au moins 20% en poids. La concentration du 5-(3-pyridylméthylène)-imidazolidine-2,4-dione dans le milieu réactionnel est généralement d'au plus 50% en poids par rapport au poids total du milieu réactionnel.

Dans une variante particulièrement préférée du procédé selon l'invention le 5-(3-pyridylméthylène)-imidazolidine-2,4-dione a été obtenu par une réaction de condensation entre le pyridine-3-carbaldéhyde et l'imidazolidine-2,4-dione.

La réaction de condensation entre le pyridine-3-carbaldéhyde et l'imidazolidine-2,4-dione est généralement effectuée en présence d'un solvant, de préférence un solvant organique.

Le cas échéant, la teneur en solvant organique dans le milieu réactionnel de la réaction de condensation est généralement d'au plus 80% en poids par rapport au poids total du milieu réactionnel. De préférence cette teneur est d'au plus 50% en poids.

Dans un premier aspect, le solvant dans la réaction de condensation est un milieu aqueux. Dans cet aspect on utilise avantageusement un catalyseur. Ce catalyseur est le plus souvent soluble dans l'eau. Un système catalytique qui donne de bons résultats comprend un acide aminé ou un sel d'acide aminé et un composé alcalin inorganique. La glycine est préférée à titre d'acide aminé. Le composé alcalin inorganique est avantageusement choisi parmi l'hydroxyde de sodium et le carbonate de sodium. Le carbonate de sodium est préféré.

Dans un deuxième aspect, le solvant dans la réaction de condensation comprend un solvant organique polaire. Le solvant organique polaire présente généralement un moment dipolaire supérieur à 2 Debye. Sont utilisables notamment des solvants capables de former des liaisons hydrogène, tels que les polyalcohols, les solvants comprenant au moins un groupement amido ou les sulfones et sulfoxides. Citons à titre d'exemple le N,N-diméthylformamide, le N-méthylpyrrolidone, le tétraméthylènesulfone l'éthylèneglycol, la glycérine et leurs mélanges. Des mélanges de N,N-diméthylformamide avec la glycérine donnent de bons résultats.

Dans un troisième aspect, le solvant dans la réaction de condensation comprend un solvant organique de faible viscosité. La viscosité du solvant organique de faible viscosité est généralement inférieure ou égale à 10 mPa.s à 25°C. Une viscosité inférieure ou égale à 5 mPa.s à 25°C convient bien. Une viscosité inférieure ou égale à 1 mPa.s à 25°C est préférée.

Dans un quatrième aspect, le solvant dans la réaction de condensation comprend un solvant organique de faible polarité. Le moment dipolaire solvant organique de faible polarité est généralement d'au plus 2 Debye. Un moment dipolaire inférieur ou égal à 1.8 Debye convient bien. Un moment dipolaire inférieur ou égal à 1 Debye est préféré.

Il a été trouvé de manière surprenante, que la mise en oeuvre d'un solvant organique de faible viscosité et/ou de faible polarité dans la réaction de condensation, permet l'obtention d'un rendement élevé en 5-(3-pyridylméthylène)-imidazolidine-2,4-dione dans des conditions de mise en oeuvre aisées, utilisables pour une synthèse industrielle du 5-(3-pyridylméthylène)-imidazolidine-2,4-dione.

Des solvants organiques particulièrement intéressants à titre de solvant organique de faible viscosité et/ou polarité sont choisis parmi les dialkyléthers linéaires, branchés ou cycliques, les esters carboxyliques et les composés aromatiques. Citons à titre d'exemple la tétrahydrofuranne, l'acétate d'éthyle, le benzène, le toluène et les xylènes. Le toluène donne de bons résultats.

La réaction de condensation entre le pyridine-3-carbaldéhyde et l'imidazolidine-2,4-dione est effectuée, de préférence, en présence d'un catalyseur de condensation. Des catalyseurs de condensation utilisables sont, par exemple, des bases telles qu'en particulier des bases azotées ou leurs sels. Souvent, le catalyseur est une base azotée organique par exemple choisie parmi les alkylamines primaires, secondaires ou tertiaires. Des amines cycliques secondaires ou leurs sels donnent de bons résultats. Citons, à titre d'exemple les dérivés de la morpholine, de la pyrrolidine et de la pipéridine. Parmi ces catalyseurs, un sel de pipéridinium est préféré. L'acétate de pipéridinium convient particulièrement bien à titre de catalyseur de condensation.

La température de la réaction de condensation entre le pyridine-3-carbaldéhyde et l'imidazolidine-2,4-dione est généralement d'au moins 50°C. De préférence la température est d'au moins 60°C. La température de la réaction de condensation entre le pyridine-3-carbaldéhyde et l'imidazolidine-2,4-dione est généralement d'au plus 200°C. De préférence la température est d'au plus 150°C. Une température d'au plus 130°C est plus particulièrement préférée.

Dans le procédé selon l'invention la réaction de condensation est généralement effectuée à un pH d'au moins 7. Le pH est de préférence d'au moins 8. Dans le procédé selon l'invention la réaction de condensation est généralement effectuée à un pH d'au plus 12. Le pH est de préférence d' au plus 10.

Dans le procédé selon l'invention, l'hydrolysa est effectuée par réaction avec une hydantoïnase, en particulier une D-hydantoïnase. Dans ce cas, la (3-pyridyl)-alanine obtenue est la D-(3-pyridyl)-alanine. Des informations concernant cette réaction sont contenues dans la demande de brevet EP1083170-A1 citée plus haut.

La décarbamatation peut être effectuée, par exemple, par nitrosation.

La (3-pyridyl)-alanine, en particulier la D-(3-pyridyl)-alanine obtenu selon le procédé selon l'invention, peut être utilisée pour la fabrication d'un peptide. Des techniques conventionnelles de couplage peptidique sont utilisables pour la fabrication du peptide.

Les exemples ci-après entendent illustrer l'invention sans toutefois la limiter.

### Exemple 1 - Synthèse de 5-(3-pyridylméthylène)-imidazolidine-2,4-dione

On a introduit 0,1 mole d'imidazolidine-2,4-dione, 3 ml d'acide acétique glacial, 0,12 mole de pyridine-3-carbaldéhyde et 30 ml de toluène dans un ballon. A ce mélange, on a rajouté 5 ml de pipéridine. L'ensemble a été chauffé au reflux pendant 17 h. Après refroidissement on a ajouté de l'isopropanol. Le précipité obtenu a été filtré et lavé à l'isopropanol. Le rendement en 5-(3-pyridylméthylène)-imidazolidine-2,4-dione était de 85%.

Le produit obtenu était directement utilisable pour la mise en oeuvre dans la réaction d'hydrogénation sans épuration supplémentaire.

### Exemple 2 - Synthèse de DL-5-(3-pyridylméthyl)-imidazolidine-2,4-dione

On a introduit de 0,13 mole de 5-(3-pyridylméthylène)-imidazolidine-2,4-dione obtenu conformément au procédé de l'exemple 1, 250 ml d'acide chlorhydrique 1N, 200 ml de méthanol et 3,26 g d'un catalyseur constitué de palladium sur charbon actif (concentration en Pd 10% en poids). On a effectué la réaction d'hydrogénation à température ambiante sous une pression initiale d'hydrogène de 2,1 bar pendant 15 h. Le milieu réactionnel a été filtré et le DL-5-(3-pyridylméthyl)-imidazolidine-2,4-dione brut obtenu après évaporation a été cristallisé avec un mélange 1:1 de méthanol :éther de méthyle tert.butylique.

### Rendement 79%

Le DL-5-(3-pyridylméthyl)-imidazolidine-2,4-dione obtenu était directement utilisable pour la mise en oeuvre dans une réaction d'hydrolyse sans épuration supplémentaire. Il était en particulier adapté pour la mise en oeuvre dans une réaction d'enzymolyse. Le procédé selon l'invention permet d'accéder au DL-5-(3-pyridylméthyl)-imidazolidine-2,4-dione de manière simple et industrialisable et avec un rendement élévé.

## Revendications

1. - Procédé de synthèse de (3-pyridyl)-alanine dans lequel
(a) on fabrique du 5-(3-pyridylméthyl)-imidazolidine-2,4-dione par une réaction d'hydrogénation avec de l'hydrogène de 5-(3-pyridylméthylène)-imidazolidine-2,4-dione dissout dans une solution aqueuse d'acide en présence d'un catalyseur d'hydrogénation;
(b) on soumet du 5-(3-pyridylméthyl)-imidazolidine-2,4-dione obtenu à une réaction d'hydrolyse avec une hydantoïnase suivie d'une réaction de décarbamatation.

2. - Procédé selon la revendication 1, dans lequel la réaction d'hydrogénation est effectuée en phase liquide.

3. - Procédé selon la revendication 1 ou 2, dans lequel le catalyseur d'hydrogénation est choisi parmi les métaux du groupe VIII du Tableau périodique des éléments.

4. - Procédé selon la revendication 3, dans lequel le catalyseur d'hydrogénation comprend du palladium.

5. - Procédé selon la revendication 4, dans lequel le catalyseur est du palladium supporté.

6. - Procédé selon l'une quelconque des revendications 1 à 5, dans lequel la température de la réaction d'hydrogénation est de -10°C à +100°C.

7. - Procédé selon l'une quelconque des revendications 1 à 6, dans lequel la pression de la réaction d'hydrogénation est de 1 bar à 15 bar.

8. - Procédé selon l'une quelconque des revendications 1 à 7, dans lequel le rapport molaire entre l'hydrogène et le 5-(3-pyridylméthylène)-imidazolidine-2,4-dione est de 1 à 1000.

9. - Procédé selon l'une quelconque des revendications 1 à 8, dans lequel la concentration du 5-(3-pyridylméthylène)-imidazolidine-2,4-dione dans le milieu réactionnel de la réaction d'hydrogénation est de 5% en poids à 50% en poids par rapport au poids total du milieu réactionnel.

10. - Procédé selon l'une quelconque des revendications 1 à 9 comprenant en outre la fabrication de 5-(3-pyridylméthylène)-imidazolidine-2,4-dione par une réaction de condensation entre le pyridine-3-carbaldéhyde et l'imidazolidine-2,4-dione.

11. - Procédé selon la revendication 10, dans lequel la réaction entre le pyridine-3-carbaldéhyde et l'imidazolidine-2,4-dione est effectuée en présence d'un catalyseur de condensation.

12. - Procédé selon la revendication 11 dans lequel le catalyseur de condensation est un sel de pipéridinium.

13. - Procédé selon l'une quelconque des revendications 10 à 12 dans lequel la température de la réaction de condensation est de 50°C à 200°C.

14. - Procédé selon l'une quelconque des revendications 10 à 13 dans lequel la réaction de condensation est effectuée à un pH de 7 à 12.

15. - Procédé selon l'une quelconque des revendications 10 à 14 dans lequel la réaction de condensation est effectuée en présence d'un solvant organique.

16. - Procédé selon l'une quelconque des revendications 1 à 15 dans lequel l'hydantoïnase est une D-hydantoïnase.

17. - Procédé selon l'une quelconque des revendications 1 à 16 dans lequel la décarbamatation est effectuée par nitrosation.

18. - Procédé selon l'une quelconque des revendications 1 à 17, dans lequel la (3-pyridyl)-alanine obtenue est la D-(3-pyridyl)-alanine.

19. - Procédé de fabrication d'un peptide comprenant
(a) l'obtention de la (3-pyridyl)-alanine selon le procédé selon l'une quelconque des revendications 1 à 18,
(b) un couplage peptidique dans lequel on utilise la (3-pyridyl)-alanine obtenue à l'étape (a).

## Claims

1. - Process for the synthesis of (3-pyridyl)alanine, in which
(a) 5-(3-pyridylmethyl)imidazolidine-2,4-dione is manufactured by hydrogenation reaction with hydrogen of 5-(3-pyridylmethylene)imidazolidine-2,4-dione dissolved in an aqueous acid solution in the presence of a hydrogenation catalyst;
(b) 5-(3-pyridylmethyl)imidazolidine-2,4-dione obtained is subjected to a hydrolysis reaction, followed by a decarbamation reaction.

2. - Process according to Claim 1, in which the hydrogenation reaction is carried out in the liquid phase.

3. - Process according to Claim 1 or 2, in which the hydrogenation catalyst is chosen from the metals from Group VIII of the Periodic Table of the Elements.

4. - Process according to Claim 3, wherein the catalyst comprises palladium.

5. - Process according to Claim 4, in which the catalyst is supported palladium.

6. - Process according to any one of Claims 1 to 5, in which the temperature of the reaction is from -10°C to +100°C.

7. - Process according to any one of Claims 1 to 6, in which the pressure of the reaction is from 1 bar to 15 bar.

8. - Process according to any one of Claims 1 to 7, in which the molar ratio of hydrogen to 5-(3-pyridylmethylene)imidazolidine-2,4-dione is from 1 to 1 000.

9. - Process according to any one of Claims 1 to 8, in which the concentration of 5-(3-pyridylmethylene)imidazolidine-2,4-dione in the reaction medium is from 5 % by weight to 50 % by weight with respect to the total weight of the reaction medium.

10. - Process according to any one of Claims 1 to 9, additionally comprising the manufacture of 5-(3-pyridylmethylene)imidazolidine-2,4-dione by a condensation reaction between pyridine-3-carbaldehyde and imidazolidine-2,4-dione.

11. - Process according to Claim 10, in which the reaction between pyridine-3-carbaldehyde and imidazolidine-2,4-dione is carried out in the presence of a condensation catalyst.

12. - Process according to Claim 11, in which the condensation catalyst is a piperidinium salt.

13. - Process according to any one of Claims 10 to 12, in which the temperature of the condensation reaction is from 50°C to 200°C.

14. - Process according to any one of Claims 10 to 13, in which the condensation reaction is carried out at a pH of 7 to 12.

15. - Process according to any one of Claims 10 to 14, wherein the condensation reaction is carried out in the presence of an organic solvent.

16. - Process according to any one of Claims 1 to 15, in which the hydrolysis is carried out by reaction with a hydantoinase.

17. - Process according to any one of Claims 1 to 16, wherein the decanbamation is carried out by nitrosation.

18. - Process according to any one of Claims 1 to 17, in which the (3-pyridyl)alanine obtained is D-(3-pyridyl)alanine.

19. - Process for the manufacture of a peptide, comprising
(a) obtaining (3-pyridyl)alanine according to the process according to any one of Claims 1 to 18,
(b) a peptide coupling, wherein the (3-pyridyl)alanine obtained is used.

## Patentansprüche

1. Verfahren zur Synthese von (3-Pyridyl)alanin, bei dem man :
(a) durch Hydrierung von in wäßriger Säurelösung gelöstem 5-(3-Pyridylmethylen)-imidazolidin-2,4-dion mit Wasserstoff in Gegenwart eines Hydrierkatalysators 5-(3-Pyridylmethyl)-imidazolidin-2,4-dion herstellt;
(b) das erhaltene 5-(3-Pyridylmethyl)-imidazolidin-2,4-dion einer Hydrolysereaktion mit einer Hydantoinase und dann einer Decarbamoylierung unterwirft.

2. Verfahren nach Anspruch 1, bei dem man die Hydrierungsreaktion in flüssiger Phase durchführt.

3. Verfahren nach Anspruch 1 oder 2, bei dem man den Hydrierkatalysator unter den Metallen der Gruppe VIII des Periodensystems der Elemente auswählt.

4. Verfahren nach Anspruch 3, bei dem der Hydrierkatalysator Palladium umfaßt.

5. Verfahren nach Anspruch 4, bei dem es sich bei dem Katalysator um geträgertes Palladium handelt.

6. Verfahren nach einem der Ansprüche 1 bis 5, bei dem die Hydriertemperatur -10°C bis +100°C beträgt.

7. Verfahren nach einem der Ansprüche 1 bis 6, bei dem der Druck der Hydrierungsreaktion 1 bar bis 15 bar beträgt.

8. Verfahren nach einem der Ansprüche 1 bis 7, bei dem das Molverhältnis von Wasserstoff zu 5-(3-Pyridylmethylen)-imidazolidin-2,4-dion 1 bis 1000 beträgt.

9. Verfahren nach einem der Ansprüche 1 bis 8, bei dem die Konzentration von 5-(3-Pyridylmethylen)-imidazolidin-2,4-dion in dem Reaktionsmedium der Hydrierreaktion 5 Gew.-% bis 50 Gew.-%, bezogen auf das Gesamtgewicht des Reaktionsmediums, beträgt.

10. Verfahren nach einem der Ansprüche 1 bis 9, bei dem man ferner 5-(3-Pyridylmethylen)-imidazolidin-2,4-dion durch eine Kondensationsreaktion zwischen Pyridin-3-carbaldehyd und Imidazolidin-2,4-dion herstellt.

11. Verfahren nach Anspruch 10, bei dem man die Reaktion zwischen Pyridin-3-carbaldehyd und Imidazolidin-2,4-dion in Gegenwart eines Kondensationskatalysators durchführt.

12. Verfahren nach Anspruch 11, bei dem es sich bei dem Kondensationskatalysator um ein Piperidiniumsalz handelt.

13. Verfahren nach einem der Ansprüche 10 bis 12, bei dem die Temperatur der Kondensationsreaktion 50°C bis 200°C beträgt.

14. Verfahren nach einem der Ansprüche 10 bis 13, bei dem man die Kondensationsreaktion bei einem pH-Wert von 7 bis 12 durchführt.

15. Verfahren nach einem der Ansprüche 10 bis 14, bei dem man die Kondensationsreaktion in Gegenwart eines organischen Lösungsmittels durchführt.

16. Verfahren nach einem der Ansprüche 1 bis 15, bei dem es sich bei der Hydantoinase um eine D-Hydantoinase handelt.

17. Verfahren nach einem der Ansprüche 1 bis 16, bei dem man die Decarbamoylierung durch Nitrosierung durchführt.

18. Verfahren nach einem der Ansprüche 1 bis 17, bei dem es sich bei dem erhaltenen 3-(Pyridyl)alanin um D-(3-Pyridyl)alanin handelt.

19. Verfahren zur Herstellung eines Peptids, bei dem man
(a) (3-Pyridyl)alanin nach dem Verfahren gemäß einem der Ansprüche 1 bis 18 herstellt,
(b) unter Verwendung des in Schritt (a) erhaltenen (3-Pyridyl)alanins eine Peptidkupplung durchführt.
